# EUROPEAN PATENT SPECIFICATION

(11) **EP 3 339 308 B1**
(45) Date of publication and mention of the grant of the patent: **27.02.2019**
(21) Application number: 15901557.7
(22) Date of filing: 11.09.2015
(51) Int. Cl.: C07D 417/06, A61K 51/04, A61K 101/02

(54) **RADIOISOTOPE FLUORINE-18 SUBSTITUTED THIAMINE, SYNTHESIS METHOD THEREFOR, AND APPLICATIONS THEREOF**
RADIOISOTOP-FLUOR-18-SUBSTITUIERTES THIAMIN, SYNTHESEVERFAHREN DAFÜR UND ANWENDUNGEN DAVON
THIAMINE À SUBSTITUTION RADIO-ISOTOPE FLUOR-18, PROCÉDÉ DE SYNTHÈSE ASSOCIÉ, ET APPLICATIONS ASSOCIÉES

(30) Priority: 17.08.2015 CN 201510504054
(43) Date of publication of application: 27.06.2018
(73) Proprietor: Zhongshan Hospital Fudan University, Xuhui, Shanghai 200032 (CN)
(72) Inventor: ZHONG, Chunjiu, Xuhui Shanghai 200032 (CN); WANG, Changpeng, Xuhui Shanghai 200032 (CN)
(74) Representative: Cabinet Beau de Loménie
(86) International application number: PCT/CN2015/089480
(87) International publication number: WO 2017/028353

(56) References cited:
- CN-A- 102 659 776
- JP-A- 2013 213 027
- US-A1- 2015 133 480
- KOLLONITSCH J ET AL: "Fluorodehydroxylation, a Novel Method for Synthesis of Fluoroamines and Fluoroamino Acids", JOURNAL OF ORGANIC CHEMISTRY,, vol. 44, no. 5, 1 March 1979 (1979-03-01), pages 771-777, XP008143751, ISSN: 0022-3263, DOI: 10.1021/JO01319A023
- GORDON LOWE ET AL: "Bacteriostatic Activity of Fluoro-analogues of 5-(2-Hydroxyethyl)-4- methylthiazole, a Metabolic Intermediate in the Biosynthesis of Thiamine", JOURNAL OF THE CHEMICAL SOCIETY, PERKIN TRANSACTIONS 1, no. 9, 1 January 1980 (1980-01-01), pages 2026-2028, XP055483670, GB ISSN: 0300-922X
- YING LI: "18F-labeling techniques for positron emission tomography", SCIENCE CHINA CHEMISTRY; THE FRONTIERS OF CHEMICAL BIOLOGY AND SYNTHESIS, vol. 56, no. 12, 31 October 2013 (2013-10-31), pages 1682-1692, XP055484012, Sience China Press ISSN: 1674-7291, DOI: 10.1007/s11426-013-5004-8
- DOI, H. ET AL.: 'Synthesis of 11C-Labeled Thiamine and Fursultiamine for in Vivo Molecular Imaging of Vitamin B1 and Its Prodrug Using Positron Emission Tomography' J. ORG. CHEM. vol. 80, no. 12, 18 May 2015, pages 6250 - 6258, XP055365127
- LI, WENXIA ET AL.: 'Vitamin B1 Deficiency and Alzheimer's Disease' CHINESE BULLETIN OF LIFE SCIENCES vol. 25, no. 2, 28 February 2013, pages 184 - 190, XP009504932
- HUANG, HUAFAN ET AL.: 'New Methods and Techniques for 18F-Labled Radiopharmaceuticals' PROGRESS IN CHEMISTRY vol. 23, no. 7, 31 July 2011, pages 1501 - 1506, XP009504933

## Description

### BACKGROUND

### Technical Field

The present invention relates to a thiamine labelled by radioisotope ¹⁸F, and synthesis method, and use thereof, and particularly to a fluorine substituted thiamine that is a thiamine derivative, synthesized by substituting the hydroxyl in the hydroxyethyl on the thiazole cycle of thiamine molecules with the radioisotope ¹⁸F, which belongs to the technical field of radiochemicals.

### Related Art

(I) Alzheimer's disease (AD) is pathophysiologically characterized by deposition of beta-amyloid (A β) protein, hyperphosphorylation of Tau protein and decreased glucose metabolism in the brain. It is the most common neurodegenerative disease and causes serious harm to individuals, families and society. So far, the diagnosis of AD depends virtually on clinical symptoms, and we lack of effective diagnostic biomarkers and detection methods. At present, there are five diagnostic biomarkers as well as related detection methods for AD (Jack CJ, et al. 2013. Acta Neuropathol.), including detection of Aβ deposition by Positron Emission Computed Tomography (PET) imaging, determination of Aβ₄₂ in cerebrospinal fluid (CSF) by enzyme linked immunosorbent assay (ELISA), determination of total tau protein/phosphorylated Tau protein in CSF by ELISA, measurement of glucose metabolism in brain by PET imaging, and detection of brain atrophy by structural magnetic resonance imaging. However, due to the limitations from the unsatisfied sensitivity/specificity of these biomarkers or the invasive and complex detection means, the new guideline issued by National Institute on Aging-Alzheimer's Association recommended these biomarkers only for research, rather than for clinical applications (McKhann GM, et al.2011. Alzheimer's & dementia).

The development of PET as well as its application in the field of AD diagnosis is very rapid. The most widely used PET tracer for AD diagnosis in clinical trials is Pittsburgh Compound B (PiB), which labelled by ¹¹C. Hence it has the inherent shortcoming that the half-life is too short (20 minutes). In recent years, the FDA of United States has successively approved three ¹⁸ F-labeled compounds for the diagnosis of AD (Machulla HJ. 2015. J Nucl Med). However, the common imaging substrate of the three compounds, Aβ deposits, has no correlation with the development of the disease (Sperling RA, et al. 2011. Alzheimer's & dementia). Therefore, the development of novel PET tracers will contribute to the clinical diagnosis of AD.

In recent years, many studies have found that the decrease in cerebral glucose metabolism is an important preclinical pathophysiological feature of AD, which occurs decades than the onset of clinical symptoms and is closely correlated with the level of cognitive function after the onset of disease (de Leon MJ, et al. 2001 PNAS; Jack CJ, et al. 2010. Lancet Neurol). By detecting cerebral glucose metabolism using 2-[¹⁸F]fluoro-2-deoxy-D-glucose (FGD) as a PET tracer, the sensitivity/specificity in diagnosis of mild AD can be up to 90%/89% (Bloudek LM, et al. 2011. J Alzheimers Dis.), and the predictive accuracy for development of amnestic mild cognitive impairment (preclinical phase of AD) into AD is as high as 84% (Mosconi L, et al. 2004. Neurology), thus showing good diagnostic value. However, the results of FDG-PET imaging of the brain in patients with AD and pre-clinical AD have complex spatial distribution characteristics in different brain regions, requiring a sophisticated skill of the clinicians.

Further studies have shown that the decrease in cerebral glucose metabolism in patients with AD is not due to the overall functional abnormalities of intracellular glucose metabolism pathway, but the three key enzymes with diphosphate thiamine (TDP, the active form of thiamine) as a common coenzyme whose activities decreased most significantly. These three key enzymes, including α-ketoglutarate dehydrogenase (KGDH), pyruvate dehydrogenase (PDH) and transketolase (TK), are involved in the only two pathways of intracellular glucose metabolism, that is, oxidative phosphorylation and pentose phosphate pathways, and the decrease in their activities will severely damage the intracellular glucose and energy metabolism, and the synthesis of neurotransmitters and substances essential for cell metabolism (such as acetylcholine, acetyl CoA, NDAH, NADPH, and ribose 5-phosphate, etc.), and lead to oxidative stress and other pathophysiological changes. Moreover, the decrease in the activities of these three enzymes is closely related to the decline of the cognitive function and the Aβ deposition in patients with AD (Gibson GE, et al. 1988. Arch Neurol; Sheu KF, et al. 1988. Arch Neurol; Bubber P, et al. 2005. Ann Neurol). TDP, as a common coenzyme of these three key enzymes, plays an important role in the normal operation of these three enzymes and the intracellular glucose metabolism pathway.

Numerous studies have shown that the mitochondrial damage, abnormal neuronal cell energy metabolism, oxidative stress, selective loss of neurons in different brain regions, abnormal phosphorylation of tau protein in the hippocampus and adjacent brain regions, increased Aβ secretion and abnormal deposition, and other pathophysiological changes induced by the decreased activities of the enzymes such as PDH and KGDH caused by dysfunctions of thiamine metabolism not only highly resemble the pathophysiology of AD, but specifically affect the cognition-related neural loops (Gibson GE, et al. 2007. Antioxid Redox Signal; Calingasan NY, et al. 1995. Brain Res.; Rao VL, et al; 1995. Alcohol Clin Exp Res). Autopsy studies from patients with AD found that the patients had thiamine deficiency and abnormal thiamine-dependent glucose metabolism, the activities of thiamine-dependent key enzymes in glucose metabolism (PDH, KGDH and TK) decreased in multiple brain regions of patients, and this decrease is not due to the reduction in the number of neurons caused by brain atrophy (Gibson GE, et al. 1988. Arch Neurol.; and Heroux M, et al. 1996. Metab Brain Dis). In addition, it was found by the present inventors in a large sample cross-section study that TDP level in AD patients is significantly lower than that in normal control. The present inventors established a novel diagnostic biomarker for AD by means of measuring the levels of thiamine and its phosphate ester in periphery blood, with sensitivity and specificity high up to 80.2% and 87.2%, respectively (Pan X, et al. 2015. EBioMedicine). *In vitro* cell experiments and brain slices from different species (including mice, cynomolgus monkeys, and humans) showed that thiamine metabolism is specifically concentrated in neurons, rather than in astrocytes; and thiamine deficiency specifically affects the neuronal ATP synthesis.

Accordingly, the inventors believe that thiamine metabolism is neuron-specific rich in the brain, and abnormal thiamine metabolism may be the cause of abnormal cerebral glucose metabolism in patients with AD and is involved in the pathogenesis of AD. The detection of cerebral thiamine metabolism may be of greater diagnostic value than the detection of cerebral glucose metabolism or blood thiamine metabolism.
(II) Glucose metabolism is enriched in various tissues and organs of human, which impairs the FDG-PET imaging of tumors. Therefore, before examination by FDG-PET imaging, the patients have to fast to lower the glucose level of peripheral blood, thereby reducing the background value of glucose metabolism in tissues and organs, and enhancing the signal to noise ratio of the tumor relative to normal tissues. Patients with diabetes are hard to receive FDG-PET examination because of the difficulties in controlling blood glucose level. In the nervous system, missed diagnosis of small focal tumors by FDG-PET imaging takes place due to the high background value of vigorous glucose metabolism; and it is difficult to distinguish tumors from inflammatory tissues where the glucose metabolism is as vigorous as in the tumor through FDG-PET examination. After injection of FDG, the patients need to rest still for one hour, otherwise the imaging may be affected because of the too much absorption of FDG into the muscle tissue due to the motion. Therefore, the examination cannot be performed with patients who cannot cooperate due to mental symptoms or other reasons.

The characteristic of high background value of glucose metabolism in tissues and organs, especially in inflammatory tissues limits the use of FDG-PET imaging for tumor diagnosis. TDP is not only in the brain as described in the background (1), but is a common coenzyme of three key enzymes of KGDH, PDH and TK in the glucose metabolism pathways of various tissues and organs of the body. The activities of the key enzymes determine the degree of glucose metabolism, and thiamine metabolism affects the activities of the three key enzymes. Thus, similar to the direct detection of glucose metabolism, detection of thiamine metabolism may also be valuable in tumor diagnosis; and due to the low abundance of thiamine, it may be possible to overcome the drawbacks of FDG-PET imaging.

JP 2013213027 and Hisashi Doi et al., Journal of Organic Chemistry, 80 (12), 2015, pages 6250-6258 disclose ¹¹C-labeled thiamine and its use in PET imaging.

In summary, the following points are drawn:
1. Existing AD diagnostic biomarkers cannot well meet the requirements of clinical application. With thiamine as a diagnostic target, a radiolabeled thiamine is developed as a PET tracer may be useful in the diagnosis of AD in the future.
2. With thiamine as a diagnostic target, a radiolabeled thiamine is developed as a PET tracer, which may be useful in the diagnosis of malignant tumor in the future.

### SUMMARY

In view of the technical problems above, a radioisotope ¹⁸F substituted thiamine is synthesized as a PET tracer in the present invention, for use in future diagnosis research of AD and tumors in clinical trials.

To solve the above problems, the present invention provides a radioisotope ¹⁸F substituted thiamine or a salt thereof, which is 3-[(4-Amino-2-methylpyrimidin-5-yl)methyl]-5-(2-fluoroethyl)-4-methylthiazolium or a salt thereof, where the fluorine atom in the fluoroethyl on the thiazole ring is radioisotope ¹⁸ F. The radioisotope ¹⁸F substituted thiamine has a chemical structure of Formula (I) below:

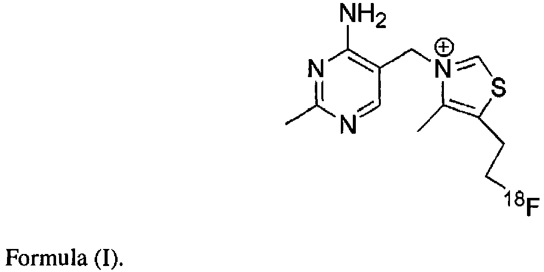

Preferably, the radioisotope ¹⁸F substituted thiamine has a chemical structure of Formula (II) or (III) below:

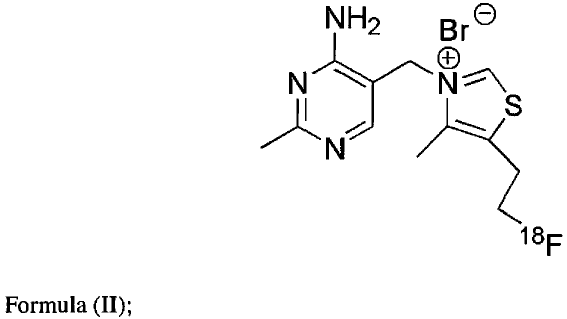

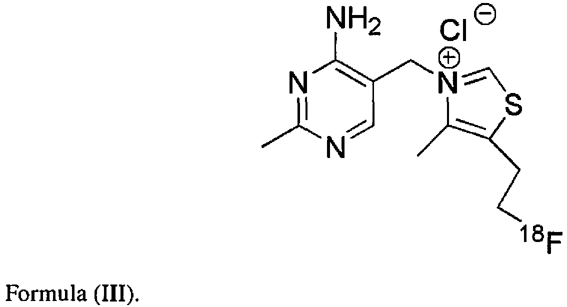

Further, the salt of the radioisotope ¹⁸F substituted thiamine is a radioisotope ¹⁸F substituted thiamine hydrobromide, having a chemical formula of C₁₂H₁₆¹⁸FN₄SBr·HBr, and a chemical structure of Formula (IV) below:

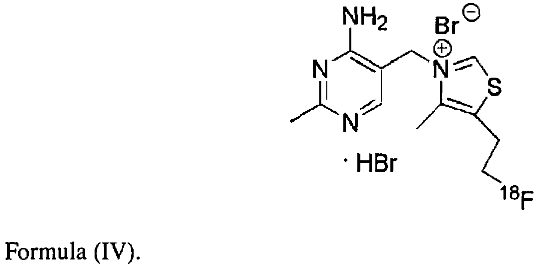

The present invention further provides a method for synthesizing the radioisotope ¹⁸F substituted thiamine hydrobromide, which comprises:
a preparation step, in which
precursors 2-(4-methylthiazol-5-yl)ethyl 4-methylbenzenesulfonate (Precursor I) and 2-methyl-5-bromomethyl-6-aminopyrimidine hydrobromide (Precursor II) of a ¹⁸F substituted thiamine are synthesized following the methods in existing literatures (Peng Xianfeng et. al., 2014, CN102659776B; and Cline J K, et al. 1937. Journal of the American Chemical Society);

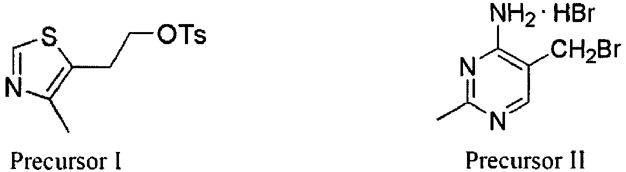

### Step 1):

generating H⁺ protons in a cyclotron, bombarding ¹⁸O-heavy oxygen water with the H⁺ protons, and generating ¹⁸F⁻ ions through the nuclear reaction ¹⁸O(p, n)¹⁸F; enriching ¹⁸F⁻ ions by a QMA column, and eluting it into a reaction flask with an eluant, to obtain [K/Kryptofix]⁺¹⁸F⁻, evaporating water to dryness; and reacting 2-(4-methylthiazol-5-yl)ethyl 4-methylbenzenesulfonate (Precursor I) with [K/Kryptofix]⁺¹⁸F⁻ to produce 5-(2-fluoroethyl)-4-methylthiazole through nucleophilic substitution in acetonitrile solvent, where the reaction equation for chemical synthesis is:

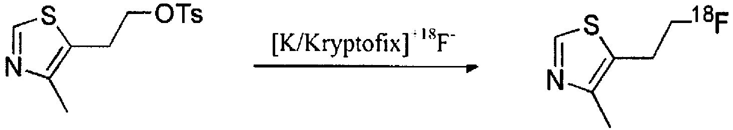

### Step 2):

reacting the intermediate product 5-(2-fluoroethyl)-4-methylthiazole obtained in Step 1) with 2-methyl-5-bromomethyl-6-aminopyrimidine hydrobromide in acetonitrile solvent to produce a ¹⁸F substituted thiamine hydrobromide, where the reaction equation for chemical synthesis is:

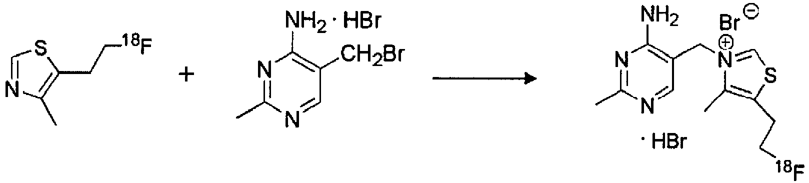

Preferably, the ratio of 2-(4-methylthiazol-5-yl)ethyl 4-methylbenzenesulfonate to the solvent in Step 1) is 5 mg:0.5-1 mL; the ratio of 2-(4-methylthiazol-5-yl)ethyl 4-methylbenzenesulfonate to ¹⁸F ions is 5 mg:4-6 Ci; and the ratio of 2-(4-methylthiazol-5-yl)ethyl 4-methylbenzenesulfonate to the eluant is 5 mg:0.6-0.9 mL, where each 0.9 mL of the eluant contains 2.52 mg KHCO₃, 9 mg Kryptofix 2.2.2, 0.72 mL acetonitrile, and 0.18 mL water.

Preferably, the ratio of 2-methyl-5-bromomethyl-6-aminopyrimidine hydrobromide to the solvent in Step 2) is 5 mg:0.5-1 mL.

Preferably, the reaction temperature in Steps 1) and 2) is 110°C.

Preferably, the mass ratio of 2-(4-methylthiazol-5-yl)ethyl 4-methylbenzenesulfonate in Step 1) to 2-methyl-5-bromomethyl-6-aminopyrimidine hydrobromide in Step 2) is 1:1.

The present invention further provides the use of the radioisotope ¹⁸F substituted thiamine in the preparation of a tracer for PET imaging of the brain in rodents.

Preferably, the rodents are C57BL/6 mice or ICR mice.

Use of the radioisotope ¹⁸F substituted thiamine in the preparation of a brain imaging agent for AD in clinic is also provided.

Use of the radioisotope¹⁸F substituted thiamine in the preparation of a PET imaging agent for tumors in clinic is further provided.

In the present invention, radioisotope ¹⁸F is used as labeling element. ¹⁸F has the advantages of long half-life (109.7 min) and small size. The introduction of a fluorine atom to a mother nucleus of thiamine with the reaction time and conditions meeting the requirements of PET imaging is very difficult. After many tests and improvements, the hydroxyl in the hydroxyethyl on the thiazole ring of thiamine molecule (structural formula: : 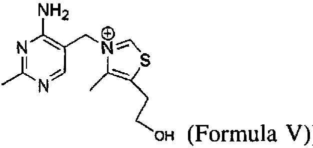 is successfully substituted with ¹⁸F atom, and a fluorine-substituted thiamine (Formula I) and a hydrobromide thereof (Formula II) are synthesized. Subsequently, the present inventors scan two strains of mice (ICR and C57BL/6) using the small animal PET/CT. The results show that ¹⁸F substituted thiamine hydrobromide successfully penetrates the blood-brain barrier and enters the brain. The uptake of ¹⁸F substituted thiamine hydrobromide in the brain of thiamine-deficient model mice is significantly higher than that in the control mice, indicating that the ¹⁸F substituted thiamine hydrobromide synthesized in the present invention can be used to evaluate the level of thiamine metabolism in the brain.

Compared with the prior art, the present invention has the following beneficial effects.

¹⁸F possess a relative long half-life, which has an obvious advantage over ¹¹C as a PET tracer. The small size of fluorine atoms does not affect the basic biological properties of thiamine. The present invention can be used as a PET tracer for providing a potential novel approach for the diagnosis of AD as well as tumors in the future.

The present invention is essentially distinguished over the ¹¹C labeled thiamine developed by Institute of Physical and Chemical Research, Japan in May 2015 (Doi H, et al. 2015. J Org Chem), in that 1. the labeled sites are different, where the C atom of the methyl attached to position 4 of the thiazole ring is substituted with a radioisotope by the Institute of Physical and Chemical Research, Japan, and in the present invention, the hydroxyl in the hydroxyethyl attached to position 5 of the thiazole ring is substituted with a radioisotope; and 2. the used radioisotopes are different, where the radioisotope used by the Institute of Physical and Chemical Research, Japan is ¹¹C, and in the present invention, ¹⁸F is used.

The present invention is essentially distinguished over the fluoroethylthiamine developed by Guangzhou Origmol Feed-Additive Co., Ltd. in May 2012 (CN 102659776 B), in that 1. in the present invention, the radioisotope ¹⁸F is used, and a stable isotope of fluorine is used by the Guangzhou Origmol Feed-Additive Co., Ltd.; 2. in the present invention, the radiochemical synthesis is greatly different in conditions and processes from the ordinary chemical synthesis of the Guangzhou Origmol Feed-Additive Co., Ltd, and the synthesized compounds are used in completely different areas; and 3. in the present invention, the synthesized ¹⁸F substituted thiamine has only the hydroxyl in the hydroxyethyl on the thiazole ring of thiamine substituted with a fluorine atom, with remaining structure unchanged; however, the methyl attached to position 2 of the pyrimidine ring of thiamine is replaced by an ethyl group by the Guangzhou Origmol Feed-Additive Co., Ltd.

### BRIEF DESCRIPTION OF THE DRAWINGS

Fig. 1 shows a synthesis route in Step 1.1 in Example 1;
Fig. 2 shows the result of TLC analysis in Step 1.1.1 in Example 1;
Fig. 3 shows the result of TLC analysis in Step 1.1.2.3 in Example 1;
Fig. 4 shows the result of TLC analysis in Step 1.1.2.4 in Example 1;
Fig. 5 shows a HPLC chromatogram obtained in Step 2 of Example 1;
Fig. 6 shows time-activity curves (TACs) of ¹⁸F substituted thiamine uptake in the brains of three ICR mice in a thiamine-deficient model group in Example 4;
Fig. 7 shows TACs of ¹⁸F substituted thiamine uptake in the brains of three ICR mice in a control group in Example 4;
Fig. 8 shows a representative image of the uptake in the brains of ICR mice in the thiamine-deficient model group in Example 4;
Fig. 9 shows a representative image of the uptake in the brains of ICR mice in the control group in Example 4;
Fig. 10 shows TACs of ¹⁸F substituted thiamine uptake in the brains of two C57BL/6 mice in a thiamine-deficient model group in Example 5;
Fig. 11 shows TACs of ¹⁸F substituted thiamine uptake in the brains of three C57BL/6 mice in a control group in Example 5;
Fig. 12 shows a representative image of the uptake in the brains of C57BL/6 mice in the thiamine-deficient model group in Example 5; and
Fig. 13 shows a representative image of the uptake in the brains of C57BL/6 mice in the control group in Example 5.

### DETAILED DESCRIPTION

To make the present invention more comprehensible and understandable, the present invention is described in further detail by way of preferred embodiments with reference to the accompanying drawings.

The eluant used in Examples 1-3 is formulated such that each 0.9 mL of the eluant contains 2.52 mg KHCO₃, 9 mg Kryptofix 2.2.2, 0.72 mL acetonitrile, and 0.18mL water.

### Example 1

### 1. Synthesis of radioisotope ¹⁸F substituted thiamine

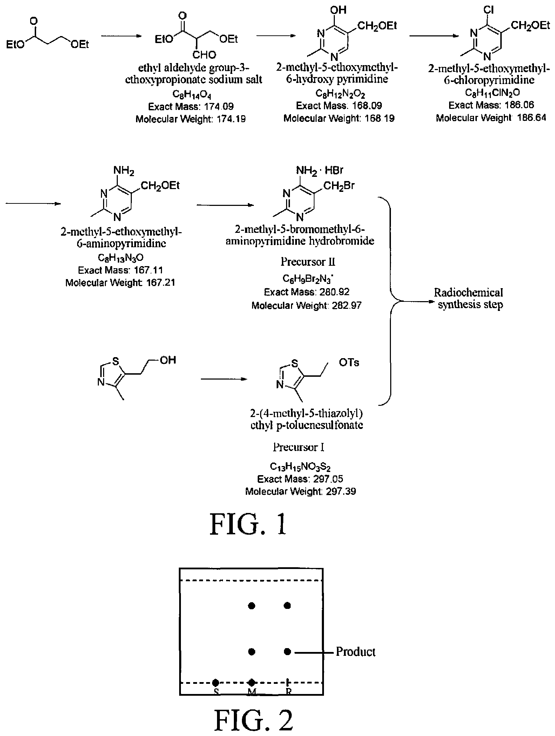

### 1.1. Synthesis of precursors (preparation step, as shown in Fig. 1)

### 1.1.1 Synthesis of precursor I: 2-(4-methylthiazol-5-yl)ethyl 4-methylbenzenesulfonate

Under nitrogen protection, 4-methyl-5-(beta-hydroxyethyl)thiazole (2.9 g, 20.0 mmol) and TsCl (19.1 g, 100 mmol) were added to a 250 mL one-neck flask, and then TEA (6.07 g, 60.0 mmol) was added, dissolved in anhydrous DCM (100 mL), and stirred for 16 hrs at normal temperature (RT). TLC analysis (n-hexane: ethyl acetate = 3: 1) was performed, as shown in Fig. 2. The resulting material was rotary evaporated, and purified by column chromatography (dichloromethane:methanol = 100 : 1), to obtain a crude product (6.1 g), which was further purified by column chromatography (dichloromethane:methanol = 80 : 1), to obtain the target product (3.2 g, yield 71%).
¹H NMR (300 MHz, CDCl₃) *δ*: 8.56 (s, 1H), 7.72 (d, 2H, *J* = 6.6 Hz), 7.31 (d, 2H, *J* = 8.1 Hz), 4.17 (t, 2H, J = 6.6 Hz), 3.12 (t, 2H, J = 6.6 Hz), 2.45 (s, 3H), 2.33 (s, 3H). MS (ESI): *m*/*z* 298 (M+1, 100).

### 1.1.2. Synthesis of precursor II: 2-methyl-5-bromomethyl-6-aminopyrimidine hydrobromide

### 1.1.2.1. Synthesis of ethyl aldehyde group-3-ethoxypropionate sodium salt

To a 500 mL three-neck flask, sodium wires (12g) soaked in anhydrous diethyl ether (200 mL) were added, and then a mixed solution containing ethyl 3-ethoxypropionate (73 g, 0.5 mol) and ethyl formate (40 g, 0.54 mol) was slowly added dropwise. The color of the reaction solution changed from colorless to yellow, and then to dark yellow. The reaction was completed within 8 hours, and stirred under RT to obtain a solid, which was directly used in the next step under nitrogen protection.

### 1.1.2.2. Synthesis of 2-Methyl-5-ethoxymethyl-6-hydroxypyrimidine

Under nitrogen protection, sodium (12 g) was fed to anhydrous ethanol (200 mL), and reacted under reflux until sodium was completely reacted, to prepare a sodium ethoxide solution. Acetamidine hydrochloride (45 g, 0.48 mol) dissolved in anhydrous ethanol (100 mL) was slowly added dropwise to a mixture of sodium ethoxide and ethyl aldehyde group-3-ethoxypropionate sodium salt, and stirred and refluxed overnight at 80°C after addition. The reaction was terminated, and adjusted to pH 6 with glacial acetic acid. The solvent was removed by rotary evaporation. Water (50 mL) was added, and extracted with DCM (2 x 100 mL). The organic phases were combined, dried over anhydrous sodium sulfate, and purified by column chromatography (methanol: dichloromethane = 1 : 100), to obtain the target product as a pale yellow solid (20.1 g, yield 24%).
¹H NMR (300 MHz, CDCl₃) *δ*: 8.02 (s, 1H), 4.40 (s, 2H), 3.62 (q, 2H, J = 6.9 Hz), 2.48 (s, 3H), 1.26 (t, 3H, *J* = 7.1 Hz). MS (ESI): *m*/*z* 169 (M+1, 100).

### 1.1.2.3. Synthesis of 2-methyl-5-ethoxymethyl-6-chloropyrimidine

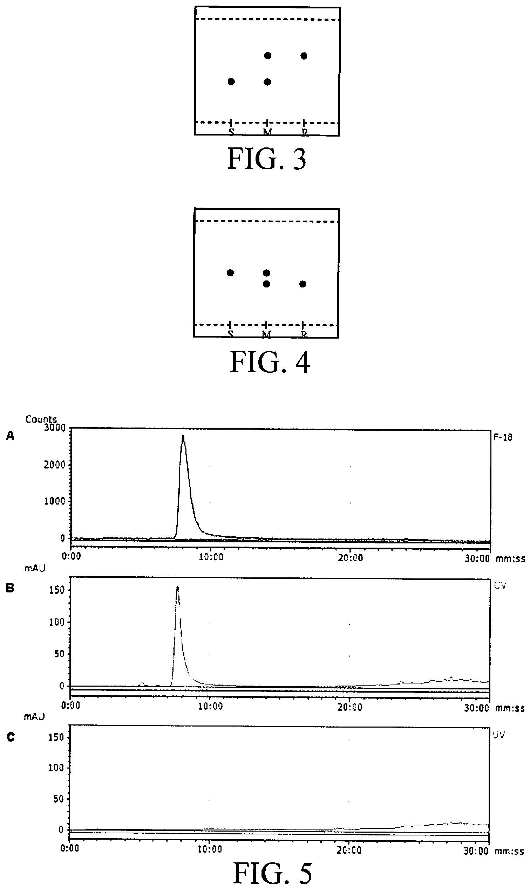

2-methyl-5-ethoxymethyl-6-hydroxypyrimidine (2 g, 12.0 mmol) was fed to a 100 mL one-neck flask, then POCl₃ (16 mL) was added and heated to 78°C, and then TEA (0.4 mL) was added dropwise. TLC analysis (methanol: dichloromethane = 1: 40) was performed 3 hrs later, as shown in Fig. 3. The reaction was terminated, and POCl₃ was removed by rotary evaporation. Water (10 mL) was added, and then NaHCO₃ was added to adjust the pH to neutral. The system was extracted with DCM (3 x 50 mL), and rotary evaporated to dryness, to obtain a crude product (2 g). The crude product was purified by column chromatography (dichloromethane : methanol = 100 : 1), to obtain a yellow oil (1.7 g, yield 77%).
¹H NMR (300 MHz, CDCl₃) *δ*: 8.64 (s, 1H), 4.56 (s, 2H), 3.64 (q, 2H, *J* = 7.1 Hz), 2.71 (s, 3H), 1.29 (t, 3H, *J* = 6.9 Hz). MS (ESI): *m*/*z* 187 (M+1, 100).

### 1.1.2.4. Synthesis of 2-methyl-5-ethoxymethyl-6-aminopyrimidine

2-methyl-5-ethoxymethyl-6-chloropyrimidine (1.5 g, 8.1 mmol) was fed to an autoclave, and then a solution of ammonia in methanol (7.1 M, 39 mL) was added, heated to 140°C, and reacted for 4.5 hrs. TLC analysis (methanol: dichloromethane = 1: 50) was performed, as shown in Fig. 4. The reaction was terminated, rotary evaporated, and purified by chromatography on a dry-packed column (methanol : dichloromethane = 1 : 100), to obtain the target product as a white solid (1.1 g, yield 82%).
¹H NMR (300 MHz, CDCl₃) *δ*: 7.99 (s, 1H), 5.47 (s, 2H), 4.43 (s, 2H), 3.49 (q, 2H, *J* = 7.1 Hz), 2.50 (s, 3H), 1.23 (t, 3H, *J* = 7.1 Hz). MS (ESI): m/z 168 (M+1, 100).

### 1.1.2.5. Synthesis of 2-methyl-5-bromomethyl-6-aminopyrimidine hydrobromide (Precursor II)

2-methyl-5-ethoxymethyl-6-aminopyrimidine (1.1 g, 6.6 mmol) was fed to a 200 mL one-neck flask, and then a HBr solution in acetic acid (10%, 73 mL) was added, heated to 100°C, and reacted for 2 hrs. The reaction was cooled to room temperature (where during the reaction, the solid was dissolved first, and then a solid was produced gradually). The reaction solution was suctioned, washed with diethyl ether, and recrystallized twice in methanol/ anhydrous diethyl ether, to obtain a white solid (1.5 g, yield 81%).
¹H NMR (300 MHz, DMSO-*d₆*) *δ*: 9.31 (s, 1H), 8.62 (s, 1H), 8.50 (s, 1H), 4.67 (s, 2H), 2.49 (s, 3H). MS (ESI): m/z 202 (M+1, 20), 154 (M-Br+OCH₃, 100).

### 1.2. Radiochemical synthesis step:

¹⁸F substituted thiamine was synthesized through a two-step synthesis process using precursors I and II. The operation steps in radiochemical synthesis were all carried out in a closed hot cell (BBS2-V, COMECER Inc.), using RNplus synthesis module (RNplus, Synthra GmbH).

### 1.2.1 (Step 1)

H⁺ protons were generated in a cyclotron (Cyclone18 Twin, IBA Inc.). ¹⁸O-heavy oxygen water having an abundance of 98% or higher was bombarded with the accelerated protons, and ¹⁸F⁻ ions (5Ci) were generated through the nuclear reaction ¹⁸O(p, n)¹⁸F. The heavy oxygen water containing ¹⁸F ions was passed through a QMA column (Sep-Pak Light QMA Carb, Waters), and then ¹⁸F was eluted into the reaction vial 1 using 0.9 mL of an eluant (containing 2.52 mg KHCO₃, 9 mg Kryptofix 2.2.2, 0.72 mL acetonitrile, and 0.18 mL water), where Kryptofix 2.2.2, K⁺ ions, and ¹⁸F⁻ ions were complexed to form [K/Kryptofix]⁺¹⁸F⁻. Acetonitrile (1 mL) was added, and evaporated at 120°C for 3 min. A nitrogen stream was blown into the reaction vial 1 at 120°C for evaporation for further 3 min. After cooling to RT, additional acetonitrile (1 mL) was added, and evaporated to dryness at 120°C for 3 min under a stream of nitrogen. After cooling to RT, precursor I (5 mg, dissolved in 0.5 mL acetonitrile) was added, and reacted for 20 min at 110°C while the reaction vial 1 was sealed, to obtain an intermediate product 5-(2-fluoroethyl)-4-methylthiazole having a structure of: 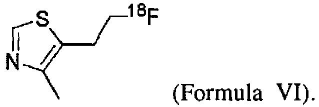 After cooling to RT, the reaction solution was stirred with a rotor for 20 s, and then transferred to a sample injector. A rinsing liquid (0.5 mL, water:acetonitrile=9:1) was further added to the reaction vial 1, stirred with a rotor for 20 s, and transferred to the sample injector. The solution (0.9 mL) was injected into a preparative column by the sample injector and separated by preparative chromatography. Pure 5-(2-fluoroethyl)-4-methylthiazole product was collected into an extraction flask (a 50 mL slender glass flask). Conditions for preparative chromatography: Waters Xbridge™ C₁₈ preparative column (19 X 100 mm, 5 um), mobile phase: 15% acetonitrile/85% water; flow rate: 14 mL/min. The desired liquid phase peak was collected based on the signal from a radio-detector, where the retention time of 5-(2-fluoroethyl)-4-methylthiazole was 10.5 min, and the collection was completed in 1 minute and 30 seconds.

### 1.2.2 (Step 2)

The preparative peak of 5-(2-fluoroethyl)-4-methylthiazole was collected in an extraction flask containing diethyl ether (20 mL) and sodium chloride (6.6 g), during which the extraction flask (sodium chloride was used to saturate water in mobile phase) was stirred with a rotor. After collection, the stirring was continued for additional 1 min, then the solution in extraction flask was stood for 1 min, and the supernatant diethyl ether phase was transferred to an intermediate flask. The diethyl ether was transferred from the intermediate flask to the reaction vial 2 where the temperature was set to 55°C, and diethyl ether was evaporated to dryness over 12 min under a stream of nitrogen. After 12 min of evaporation, acetonitrile (1 mL) was added into the reaction vial 2 to dissolve the 5-(2-fluoroethyl)-4-methylthiazole, stirred for 10 sec and then passed through a magnesium sulfate column. Additional acetonitrile (1 mL) was added to rinse the reaction vial 2 and elute the 5-(2-fluoroethyl)-4-methylthiazole off from the magnesium sulfate column. The acetonitrile solution flowing through the magnesium sulfate column was transferred back to the reaction vial 2. Precursor II (5 mg, previously dissolved in 1 mL hot acetonitrile) was added, and reacted for 20 min at 110°C while the reaction vial 2 was sealed, to obtain a ¹⁸F substituted thiamine product. At the same time, the sample injector was washed with methanol repeatedly. In the heating process during the reaction, a nitrogen stream was blown to reduce the volume of the solvent acetonitrile. When the temperature reached 110°C, the nitrogen stream was shut off, and the reaction vial 2 was sealed. After reaction, the system was maintained at 110°C for 3 min, during which a nitrogen stream was blown to contribute to evaporate the acetonitrile to dryness, and then the system was cooled to RT. After cooling to RT, water (0.5 mL) was added, stirred for 20 sec to dissolve ¹⁸F substituted thiamine, and then transferred to the sample injector for separating and collecting a pure ¹⁸F substituted thiamine product by preparative chromatography. Conditions for preparative chromatography: Waters Xbridge™ C18 preparative column (19 X 100 mm, 5 um), mobile phase: 5% methanol/95% water (containing 50 mM ammonium acetate); flow rate: 14mL/min. The desired liquid phase peak was collected based on the signal from a radio-detector, where the ¹⁸F substituted thiamine retention time was 6.5 min, and the collection was completed in 30 sec.

The whole radiochemical synthesis process is completed in about 2 hours. The radiochemical output of ¹⁸F substituted thiamine is > 75mCi, the radiochemical yield is > 3% (decay-correction to end of bombardment), the radiochemical purity is > 95% and the specific activity is > 1.5Ci/µmol.
2. The purity of the product was detected by analytical HPLC. The result is shown in Fig. 5.
2.1. Conditions for analytical chromatography:
   2.2. Analytic chromatographic column: shim-pack VP-ODS 5 uM C₁₈ (4.6 X 150mm) available from Shimadzu.
   2.3. Mobile phases: Phase A: water (with 0.05% triethylamine and 50 mM ammonium acetate); and Phase B: methanol. 0-15 min: A:B = 85%:15%; 15-25 min: Phase A gradient from 85% to 0%, and Phase B gradient from 15% to 100%; and 25-30 min: Phase B: 100%. Flow rate: 0.8 mL/min.
   2.4 Setting of radio-detection probe: ¹⁸F. Setting of UV detection light source: deuterium lamp, and detection probe: 254 nm. The radio-detection probe is located behind the UV probe, and the regular time interval is 24 seconds at 0.8ml/min flow rate.
   2.5. Other remarks

In Figs. 5A and B, the product collected in the preparation of ¹⁸F substituted thiamine was mixed with the standard sample (¹⁹F substituted thiamine), and then the mixture was loaded into analytical HPLC. A shows the radio-signal, with the retention time being 7 minutes and 59 seconds; and B shows the UV-signal, with the retention time being 7 minutes and 35 seconds. The radio-probe is located behind the UV probe, and the time delay is 24 seconds. C shows the UV signal when the ¹⁸F substituted thiamine is loaded alone.

### Example 2

### 1. Synthesis of radioisotope ¹⁸F substituted thiamine

### 1.1. Synthesis of precursors (the same as Step 1.1 in Example 1)

### 1.2. Radiochemical synthesis step:

Hot-labeled 18F substituted thiamine was synthesized through a two-step synthesis process using precursors I and II. The operation steps in radiochemical synthesis were all carried out in a closed hot cell (BBS2-V, COMECER Inc.), using RNplus synthesis module (RNplus, Synthra GmbH).

### 1.2.1 (Step 1)

H⁺ protons were generated in a cyclotron (Cyclone18 Twin, IBA Inc.). ¹⁸O-heavy oxygen water having an abundance of 98% or higher was bombarded with the accelerated protons, and ¹⁸F⁻ ions (4Ci) were generated through the nuclear reaction ¹⁸O(p, n)¹⁸F. The heavy oxygen water was passed through a QMA column (Sep-Pak Light QMA Chloride type, Waters) that was previously activated with a NaHCO₃ solution (10 mL, 0.5M) and water (10 mL), and then ¹⁸F was eluted into a reaction vial 1 using 0.6 mL of an eluant, where Kryptofix 2.2.2, K⁺ ions, and ¹⁸F ions were complexed to form [K/Kryptofix]⁺¹⁸F⁻. Acetonitrile (1 mL) was added, and evaporated at 120°C for 3 min. A nitrogen stream was introduced into the reaction vial 1 at 120°C for evaporation for further 3 min. After cooling to room temperature, additional acetonitrile (1 mL) was added, and evaporated to dryness at 120°C over 3 min under a stream of nitrogen. After cooling to room temperature, precursor I (5 mg, dissolved in 0.6 mL acetonitrile) was added, and reacted for 20 min at 110°C while the reaction flask was closed, to obtain an intermediate product 5-(2-fluoroethyl)-4-methylthiazole having a structure of: 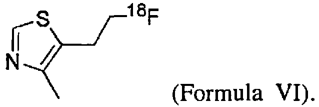 After cooling to room temperature, the reaction solution was stirred with a rotor for 20 s, and then transferred to a sample injector. A rinsing liquid (0.5 mL, water:acetonitrile=9:1) was further added to the reaction vial 1, stirred with a rotor for 20 s, and transferred to the sample injector. The solution (0.9 mL) was injected into a preparative column by the sample injector and separated by preparative chromatography. Pure 5-(2-fluoroethyl)-4-methylthiazole product was collected into an extraction flask (a 50 mL slender glass flask). Conditions for preparative chromatography: Waters Xbridge™ C₁₈ preparative column (19 X 100 mm, 5 um), mobile phase: 15% acetonitrile/85% water; flow rate: 14 mL/min. The desired liquid phase peak was collected based on the signal from a gamma detector, where the retention time of 5-(2-fluoroethyl)-4-methylthiazole was 10.5 min, and the collection was completed in 1 minute and 30 seconds.

### 1.2.2 (Step 2)

The preparative peak of 5-(2-fluoroethyl)-4-methylthiazole was collected in an extraction flask containing diethyl ether (20 mL) and sodium chloride (6.6 g), during which the extraction flask (sodium chloride was used to saturate water in mobile phase) was stirred with a rotor. After collection, the stirring was continued for additional 1 min, then the extraction flask was stood for 1 min, and the supernatant diethyl ether phase was transferred to an intermediate flask. The diethyl ether was transferred from the intermediate flask to a reaction vial 2 where the temperature was set to 55°C, and diethyl ether was evaporated to dryness over 12 min under a stream of nitrogen. After 12 min of evaporation, acetonitrile (1 mL) was added to the reaction vial 2 to dissolve the 5-(2-fluoroethyl)-4-methylthiazole, stirred for 10 sec and then passed through a magnesium sulfate column. Additional acetonitrile (1 mL) was added to rinse the reaction vial 2 and elute the 5-(2-fluoroethyl)-4-methylthiazole off from the magnesium sulfate column. The acetonitrile solution flowing through the magnesium sulfate column was transferred back to the reaction vial 2. Precursor II (5 mg, previously dissolved in 0.5 mL hot acetonitrile) was added, and reacted for 20 min at 110°C while the reaction flask was closed, to obtain a 18F substituted thiamine product. At the same time, the sample injector was washed with methanol repeatedly. In the heating process during the reaction, a nitrogen stream was introduced to reduce the volume of the solvent acetonitrile. When the temperature reached 110°C, the nitrogen stream was shut off, and the reaction vial 2 was closed. After reaction, the system was maintained at 110°C for 3 min, during which a nitrogen stream was introduced to evaporate the acetonitrile to dryness, and then the system was cooled to room temperature. After cooling to room temperature, water (0.5 mL) was added, stirred for 20 sec to dissolve ¹⁸F substituted thiamine, and then transferred to the sample injector for separating and collecting a pure ¹⁸F substituted thiamine product by preparative chromatography. Conditions for preparative chromatography: Waters Xbridge™ C18 preparative column (19 X 100 mm, 5 um), mobile phase, 5% methanol/95% water (containing 50 mM ammonium acetate); flow rate, 14mL/min. The desired liquid phase peak was collected based on the signal from a gamma detector, where the ¹⁸F substituted thiamine retention time was 6.5 min, and the collection was completed in 30 sec.

The whole radiochemical synthesis step is completed in about 2 hours. The radiochemical output of ¹⁸F substituted thiamine is > 50mCi, the radiochemical yield is > 2% (with attenuation correction), the radiochemical purity is > 95% and the specific activity is > 1Ci/µmol.
2. The purity of the product was detected by analytical HPLC (as described in Step 2 of Example 1).

### Example 3

### 1. Synthesis of radioisotope ¹⁸F substituted thiamine.

### 1.1. Synthesis of precursors (the same as Step 1.1 in Example 1)

### 1.2. Radiochemical synthesis step:

Hot-labeled ¹⁸F substituted thiamine was synthesized through a two-step synthesis process using Precursors I and II. The operation steps in radiochemical synthesis were all carried out in a closed hot cell (BBS2-V, COMECER Inc.), using RNplus synthesis module (RNplus, Synthra GmbH).

### 1.2.1 (Step 1)

H⁺ protons were generated in a cyclotron (Cyclone18 Twin, IBA Inc.). ¹⁸O-heavy oxygen water having an abundance of 98% or higher was bombarded with the accelerated protons, and ¹⁸F⁻ ions (6Ci) were generated through the nuclear reaction ¹⁸O(p, n)¹⁸F. The heavy oxygen water was passed through a QMA column (Sep-Pak Light QMA Carb, Waters), and then ¹⁸F was eluted into a reaction vial 1 using 0.8 mL of an eluant, where Kryptofix 2.2.2, K⁺ ions, and ¹⁸F⁻ ions were complexed to form [K/Kryptofixt¹⁸F⁻. Acetonitrile (1 mL) was added, and evaporated at 120°C for 3 min. A nitrogen stream was introduced into the reaction vial 1 at 120°C for evaporation for further 3 min. After cooling to room temperature, additional acetonitrile (1 mL) was added, and evaporated to dryness at 120°C over 3 min under a stream of nitrogen. After cooling to room temperature, precursor I (5 mg, dissolved in 1 mL acetonitrile) was added, and reacted for 20 min at 110°C while the reaction flask was closed, to obtain an intermediate product 5-(2-fluoroethyl)-4-methylthiazole having a structure of: 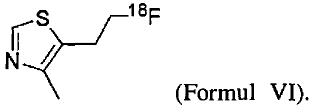 After cooling to room temperature, the reaction solution was stirred with a rotor for 20 s, and then transferred to a sample injector. A rinsing liquid (0.5 mL, water:acetonitrile=9:1) was further added to the reaction vial 1, stirred with a rotor for 20 s, and transferred to the sample injector. The solution (0.9 mL) was injected into a preparative column by the sample injector and separated by preparative chromatography. Pure 5-(2-fluoroethyl)-4-methylthiazole product was collected into an extraction flask (a 50 mL slender glass flask). Conditions for preparative chromatography: Waters Xbridge™ C₁₈ preparative column (19 X 100 mm, 5 um), mobile phase: 15% acetonitrile/85% water; flow rate: 14 mL/min. The desired liquid phase peak was collected based on the signal from a gamma detector, where the retention time of 5-(2-fluoroethyl)-4-methylthiazole was 10.5 min, and the collection was completed in 1 minute and 30 seconds.

### 1.2.2 (Step 2)

The preparative peak of 5-(2-fluoroethyl)-4-methylthiazole was collected in an extraction flask containing diethyl ether (15 mL) and sodium chloride (6.6 g), during which the extraction flask (sodium chloride was used to saturate water in mobile phase) was stirred with a rotor. After collection, the stirring was continued for additional 1 min, then the extraction flask was stood for 1 min, and the supernatant diethyl ether phase was transferred to an intermediate flask. The diethyl ether was transferred from the intermediate flask to a reaction vial 2 where the temperature was set to 55°C, and diethyl ether was evaporated to dryness over 15 min under a stream of nitrogen. After 15 min of evaporation, acetonitrile (1 mL) was added to the reaction vial 2 to dissolve the 5-(2-fluoroethyl)-4-methylthiazole, stirred for 10 sec and then passed through a magnesium sulfate column. Additional acetonitrile (1 mL) was added to rinse the reaction vial 2 and elute the 5-(2-fluoroethyl)-4-methylthiazole off from the magnesium sulfate column. The acetonitrile solution flowing through the magnesium sulfate column was transferred back to the reaction vial 2. Precursor II (5 mg, previously dissolved in 0.8 mL hot acetonitrile) was added, and reacted for 30 min at 100°C while the reaction flask was closed, to obtain a 18F substituted thiamine product. At the same time, the sample injector was washed with methanol repeatedly. In the heating process during the reaction, a nitrogen stream was introduced to reduce the volume of the solvent acetonitrile. When the temperature reached 100°C, the nitrogen stream was shut off, and the reaction vial 2 was closed. After reaction, the system was maintained at 110°C for 3 min, during which a nitrogen stream was introduced to evaporate the acetonitrile to dryness, and then the system was cooled to room temperature. After cooling to room temperature, water (0.5 mL) was added, stirred for 20 sec to dissolve ¹⁸F substituted thiamine, and then transferred to the sample injector for separating and collecting a pure ¹⁸F substituted thiamine product by preparative chromatography. Conditions for preparative chromatography: Waters Xbridge™ C18 preparative column (19 X 100 mm, 5 um), mobile phase: 5% methanol/95% water (containing 50 mM ammonium acetate); flow rate, 14mL/min. The desired liquid phase peak was collected based on the signal from a gamma detector, where the ¹⁸F substituted thiamine retention time was 6.5 min, and the collection was completed in 30 sec.

The whole radiochemical synthesis step is completed in about 2 hours. The radiochemical output of ¹⁸F substituted thiamine is > 50mCi, the radiochemical yield is > 2% (with attenuation correction), the radiochemical purity is > 95% and the specific activity is > 1Ci/µmol.
2. The purity of the product was detected by analytical HPLC (as described in Step 2 of Example 1).

### Example 4

1. Experiments on scanning of ICR mice by using ¹⁸F substituted thiamine as a PET/CT tracer.
1.1. Experimental drug: ¹⁸F substituted thiamine (synthesized and identified as in Example 1).
1.2. Animals and treatments: 8-week-old ICR mice, SPF grade, purchased from Shanghai SLAC Laboratory Animal Co., Ltd, were housed in a barrier environment and assigned to a treatment group and a control group randomly. The animals in the treatment group were fed with thiamine deficient diet (n = 3), and the animals in the control group were fed with normal diet (n = 3). After 28 days, PET/CT scan of the mice was performed.
1.3. PET/CT scan of the mice: small animal PET/CT scanner (Inveon, Siemens) thickness of PET layer: 0.78 mm, 128*128 matrix, energy window: 350-650 kev, CT tube current: 500 uA, tube voltage: 80 kv; scan mode: dynamic scan; scan time: 90 min.
1.4. Processing of PET/CT image: After the acquisition, the images were reconstructed with two iterations of 3D OSEM. The whole brain was delimited as the region of interest (ROI) by the image processing software coming with the Inveon small animal PET/CT scanner. The Standard uptake value (SUV) and TAC within 90 min were calculated.
1.5. Experimental results

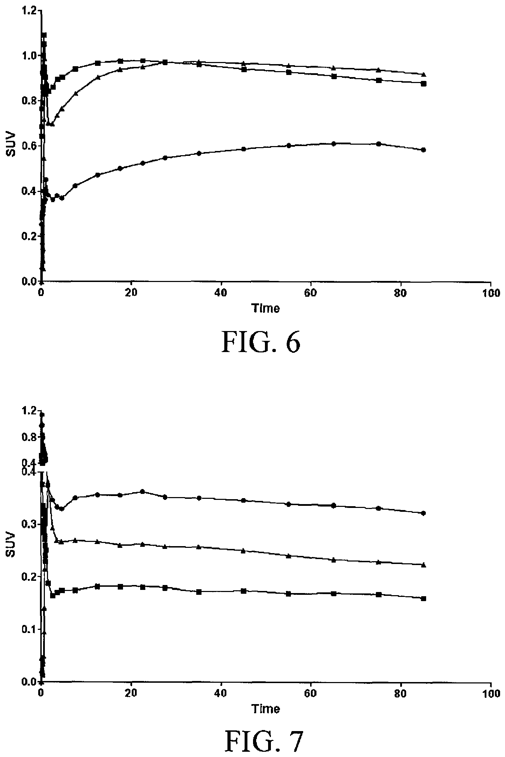

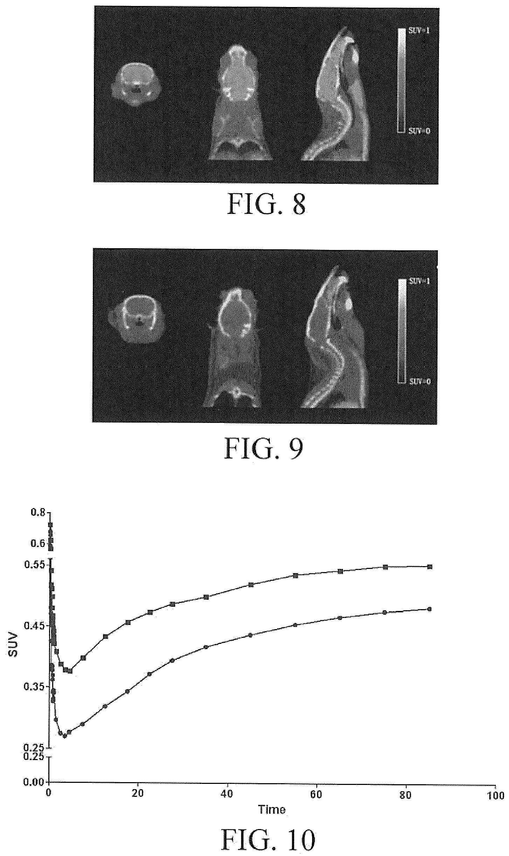

In the thiamine-deficient group, the peaks of ¹⁸F substituted thiamine absorption in the brains of two mice occurred at 22.5 and 35 minutes, respectively, at which the SUV values were 0.98 and 0.97, respectively, followed by a slow decline. The peak of ¹⁸F substituted thiamine absorption in the brain of the third mice occurred at 65 minutes, at which the SUV value was 0.61, followed by a slow decline (as shown in Fig. 6). In the control group, the absorption peaks in the brains of two mice occurred at about 20 min, at which the SUV values were 0.18 and 0.36, respectively. The SUV value in the third mice was stable within 20 min and was between 0.26 and 0.27, followed by a slow decline (as shown in Fig. 7). It can be seen from Figs. 8 and 9 that the ¹⁸F substituted thiamine uptake in the brains of thiamine-deficient group was obviously increased.

### Example 5

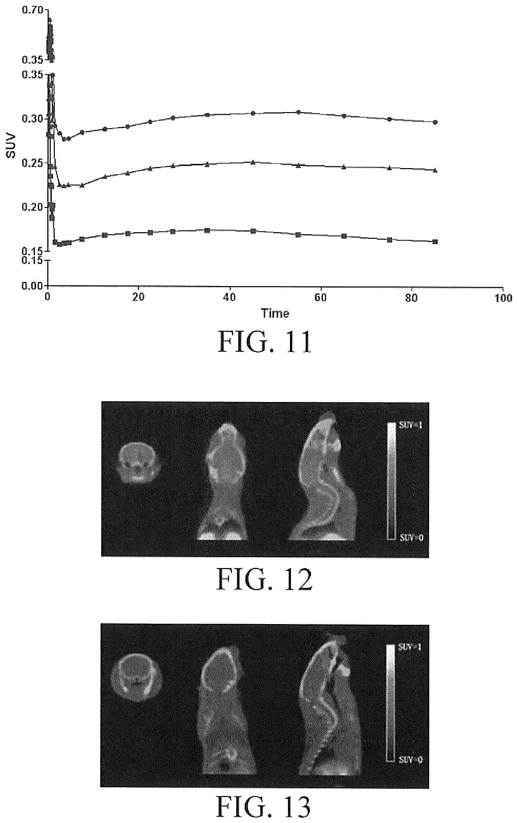

1. Experiments on scanning of C57BL/6 mice by using ¹⁸F substituted thiamine as a PET/CT tracer.
1.1. Experimental drug: ¹⁸F substituted thiamine (synthesized and identified as in Example 1).
1.2. Animals and treatments: 8-week-old C57BL/6 mice, SPF grade, purchased from Shanghai SLAC Laboratory Animal Co., Ltd, were housed in a barrier environment and assigned to a treatment group and a control group, randomly. The animals in the treatment group were fed with thiamine deficient diet (n = 2), and the animals in the control group were fed with normal diet (n = 3). After 28 days, PET/CT scan of the mice was performed.
1.3. PET/CT scan of the mice: Small animal PET/CT scanner (Inveon, Siemens), thickness of PET layer: 0.78 mm, 128*128 matrix, energy window: 350-650 kev, CT tube current: 500 uA, tube voltage: 80 kv; scan mode: dynamic scan; scan time: 90 min.
1.4. Processing of PET/CT image: After the acquisition, the images were reconstructed with two iterations of 3D OSEM. The whole brain was delimited as the region of interest (ROI) by the image processing software coming with the Inveon small animal PET/CT scanner. The SUV and TAC within 90 min were calculated.
1.5. Experimental results: In the thiamine-deficient group, the accumulation of ¹⁸F substituted thiamine in the brains of two C57BL/6 mice rose continuously over 90 min, and gradually reached a peak at 90 min, at which the SUV values are 0.48 and 0.55, respectively (as shown in Fig. 10). In the control group, the absorption peaks in the brains of three mice occurred from 35 to 55 min, at which the SUV values were 0.17, 0.25, and 0.3, respectively (as shown in Fig. 11). It can be seen from Figs. 12 and 13 that the ¹⁸F substituted thiamine uptake in the brains of thiamine-deficient C57BL/6 mice was obviously increased.

## Claims

1. A radioisotope ¹⁸F substituted thiamine or a salt thereof, which is 3-[(4-amino-2-methylpyrimidin-5-yl)methyl]-5-(2-fluoroethyl)-4-methylthiazolium or a salt thereof, where the fluorine element in the fluoroethyl on the thiazole ring is radioisotope ¹⁸F, having a chemical structure of Formula (I) below:

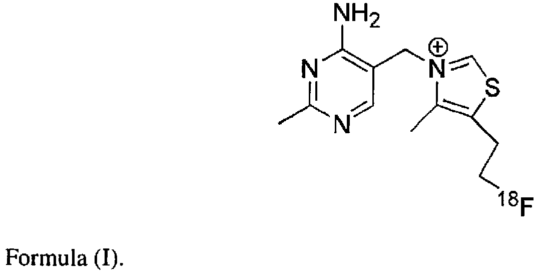

2. The radioisotope ¹⁸F substituted thiamine or a salt thereof according to claim 1, having a chemical structure of Formula (II) or (III) below:

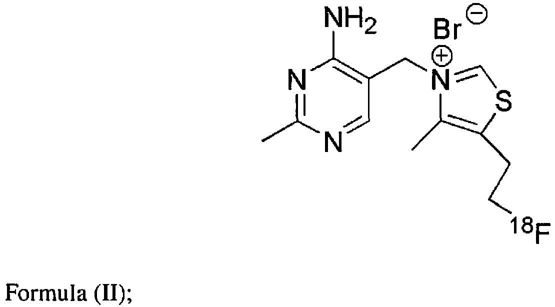

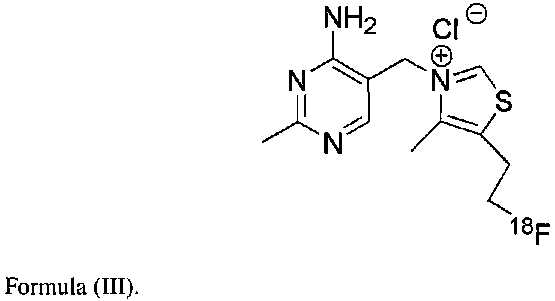

3. The radioisotope ¹⁸F substituted thiamine or a salt thereof according to claim 2, wherein the salt of the radioisotope ¹⁸F substituted thiamine is specifically a radioisotope ¹⁸F substituted thiamine hydrobromide having a chemical formula of C₁₂H₁₆¹⁸FN₄SBr·HBr, and a chemical structure of Formula (IV) below:

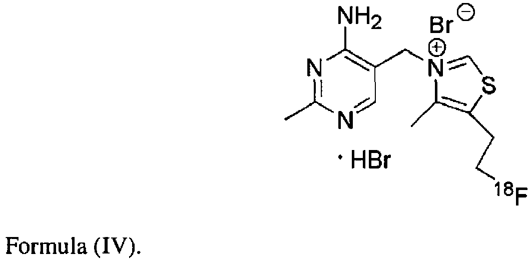

4. A method for synthesizing a radioisotope ¹⁸F substituted thiamine hydrobromide according to claim 3, comprising:
Step 1): generating H⁺ protons in a cyclotron, bombarding ¹⁸O-heavy oxygen water with the H⁺ protons, and generating ¹⁸F ions through the nuclear reaction ¹⁸O(p, n)¹⁸F; enriching ¹⁸F ions by a QMA column, and eluting it into a reaction vial with an eluant, to obtain [K/Kryptofix]⁺¹⁸F⁻, evaporating water to dryness; and reacting 2-(4-methylthiazol-5-yl)ethyl 4-methylbenzenesulfonate with [K/Kryptofix]⁺¹⁸F⁻ to produce 5-(2-fluoroethyl)-4-methylthiazole through nucleophilic substitution in acetonitrile solvent, where the reaction equation for chemical synthesis is: 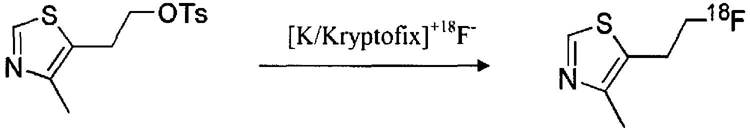
Step 2): reacting the product 5-(2-fluoroethyl)-4-methylthiazole obtained in Step 1) with 2-methyl-5-bromomethyl-6-aminopyrimidine hydrobromide in acetonitrile solvent to produce ¹⁸F substituted thiamine hydrobromide, where the reaction equation for chemical synthesis is:

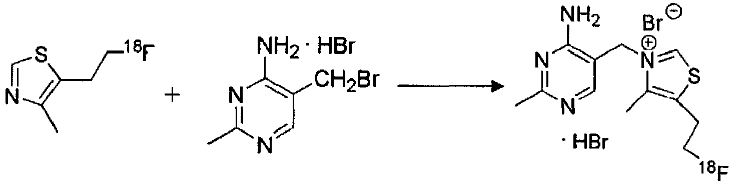

5. The method for synthesizing radioisotope ¹⁸F substituted thiamine hydrobromide according to claim 4, wherein the ratio of 2-(4-methylthiazol-5-yl)ethyl 4-methylbenzenesulfonate to the solvent in Step 1) is 5 mg:0.5-1 mL; the ratio of 2-(4-methylthiazol-5-yl)ethyl 4-methylbenzenesulfonate to ¹⁸F⁻ ions is 5 mg:4-6 Ci; and the ratio of 2-(4-methylthiazol-5-yl)ethyl 4-methylbenzenesulfonate to the eluant is 5 mg:0.6-0.9 mL, where each 0.9 mL of the eluant contains 2.52 mg KHCO₃, 9 mg Kryptofix 2.2.2, 0.72 mL acetonitrile, and 0.18 mL water.

6. The method for synthesizing radioisotope ¹⁸F substituted thiamine hydrobromide according to claim 4, wherein the ratio of 2-methyl-5-bromomethyl-6-aminopyrimidine hydrobromide to the solvent in Step 2) is 5 mg:0.5-1 mL.

7. The method for synthesizing radioisotope ¹⁸F substituted thiamine hydrobromide according to any one of claims 4 to 6, wherein the reaction temperature in Steps 1) and 2) is 110°C.

8. The method for synthesizing radioisotope ¹⁸F substituted thiamine hydrobromide according to claim 4, wherein the mass ratio of 2-(4-methylthiazol-5-yl)ethyl 4-methylbenzenesulfonate in Step 1) to 2-methyl-5-bromomethyl-6-aminopyrimidine hydrobromide in Step 2) is 1:1.

9. Use of the radioisotope ¹⁸F substituted thiamine according to claim 1 in the preparation of a tracer for PET imaging of the brain in rodents.

10. Use of the radioisotope ¹⁸F substituted thiamine according to claim 1 in the preparation of a tracer for PET imaging of clinical trials for Alzheimer's disease.

11. Use of the radioisotope ¹⁸F substituted thiamine according to claim 1 in the preparation of a tracer for PET imaging of clinical trials for tumors.

## Patentansprüche

1. Radioisotop- ¹⁸F-substituiertes Thiamin oder ein Salz davon, das 3-[(4-Amino-2-methylpyrimidin-5-yl)methyl]-5-(2-fluorethyl)-4-methylthiazolium oder ein Salz davon ist, wobei das Element Fluor im Fluorethyl am Thiazolring das Radioisotop ¹⁸F ist, mit einer chemischen Struktur der nachstehenden Formel (I):

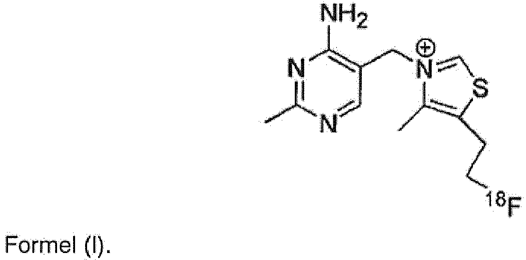

2. Radioisotop- ¹⁸F-substituiertes Thiamin oder ein Salz davon nach Anspruch 1 mit einer chemischen Struktur der nachstehenden Formel (II) oder (III):

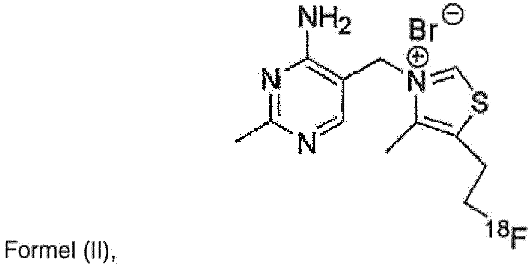

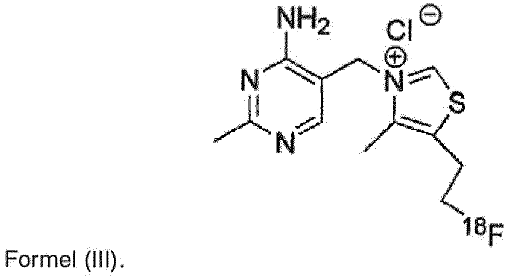

3. Radioisotop- ¹⁸F-substituiertes Thiamin oder ein Salz davon nach Anspruch 2, wobei das Salz des Radioisotop- ¹⁸F-substituierten Thiamins insbesondere ein Radioisotop- ¹⁸F-substituiertes Thiaminhydrobromid mit einer chemischen Formel C₁₂H-₁₆¹⁸FN₄SBr·HBr und einer chemischen Struktur der nachstehenden Formel (IV) ist:

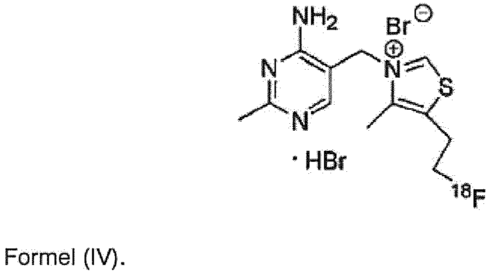

4. Verfahren zum Synthetisieren eines Radioisotop- ¹⁸F-substituierten Thiaminhydrobromids nach Anspruch 3, umfassend:
Stufe 1): Erzeugen von H⁺-Protonen in einem Zyklotron, Bombardieren von schwerem Sauerstoff- ¹⁸O-Wasser mit den H⁺-Protonen und Erzeugen von ¹⁸F⁻-Ionen durch die Kernreaktion ¹⁸O(p, n) ¹⁸F, Anreichern von ¹⁸F⁻-Ionen durch eine QMA-Säule und Eluieren dieser in ein Reaktionsgefäß mit einem Elutionsmittel, um [K/Kryptofix]⁺¹⁸F⁻ zu erhalten, Verdampfen des Wasser zur Trockne, und Umsetzen von 2-(4-Methylthiazol-5-yl)ethyl-4-methylbenzolsulfonat mit [K/Kryptofix]⁺¹⁸F⁻ zur Erzeugung von 5-(2-Fluorethyl)-4-methylthiazol durch nukleophile Substitution in einem Acetonitril-Lösungsmittel, wobei die Reaktionsgleichung für die chemische Synthese wie folgt lautet: 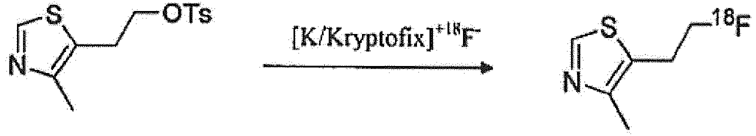
Stufe 2): Umsetzen des in Stufe 1) erhaltenen Produkts 5-(2-Fluorethyl)-4-methylthiazol mit 2-Methyl-5-brommethyl-6-aminopyrimidinhydrobromid in einem Acetonitril-Lösungsmittel zur Erzeugung von ¹⁸F-substituiertem Thiaminhydrobromid, wobei die Reaktionsgleichung für die chemische Synthese wie folgt lautet:

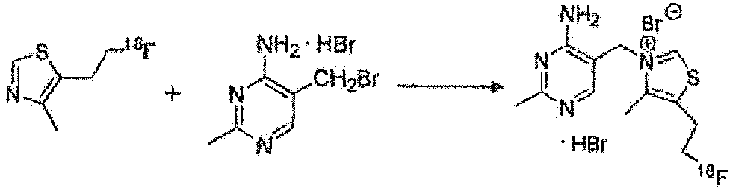

5. Verfahren zum Synthetisieren von Radioisotop- ¹⁸F-substituiertem Thiaminhydrobromid nach Anspruch 4, wobei das Verhältnis von 2-(4-Methylthiazol-5-yl)ethyl-4-methylbenzolsulfonat zu dem Lösungsmittel in Stufe 1) 5 mg:0,5-1 ml beträgt, das Verhältnis von 2-(4-Methylthiazol-5-yl)ethyl-4-methylbenzolsulfonat zu ¹⁸F⁻-Ionen 5 mg:4-6 Ci beträgt, und das Verhältnis von 2-(4-Methylthiazol-5-yl)ethyl-4-methylbenzolsulfonat zu dem Elutionsmittel 5 mg:0,6-0,9 ml beträgt, wobei jede 0,9 ml des Elutionsmittels 2,52 mg KHCO₃, 9 mg Kryptofix 2.2.2, 0,72 ml Acetonitril und 0,18 ml Wasser enthalten.

6. Verfahren zum Synthetisieren von Radioisotop- ¹⁸F-substituiertem Thiaminhydrobromid nach Anspruch 4, wobei das Verhältnis von 2-Methyl-5-brommethyl-6-aminopyrimidinhydrobromid zu dem Lösungsmittel in Stufe 2) 5 mg:0,5-1 ml beträgt.

7. Verfahren zum Synthetisieren von Radioisotop- ¹⁸F-substituiertem Thiaminhydrobromid nach einem der Ansprüche 4 bis 6, wobei die Reaktionstemperatur in den Stufen 1) und 2) 110 °C beträgt.

8. Verfahren zum Synthetisieren von Radioisotop- ¹⁸F-substituiertem Thiaminhydrobromid nach Anspruch 4, wobei das Massenverhältnis von 2-(4-Methylthiazol-5-yl)ethyl-4-methylbenzolsulfonat in Stufe 1) zu 2-Methyl-5-brommethyl-6-aminopyrimidinhydrobromid in Stufe 2) 1:1 beträgt.

9. Verwendung des Radioisotop- ¹⁸F-substituierten Thiamins nach Anspruch 1 bei der Herstellung eines Tracers für eine PET-Bildgebung des Gehirns bei Nagetieren.

10. Verwendung des Radioisotop- ¹⁸F-substituierten Thiamins nach Anspruch 1 bei der Herstellung eines Tracers für eine PET-Bildgebung klinischer Prüfungen zur Alzheimer-Krankheit.

11. Verwendung des Radioisotop- ¹⁸F-substituierten Thiamins nach Anspruch 1 bei der Herstellung eines Tracers für eine PET-Bildgebung klinischer Prüfungen zu Tumoren.

## Revendications

1. Thiamine substituée par le radioisotope ¹⁸F ou sel de celle-ci, laquelle qui est le 3-[(4-amino-2-méthylpyrimidin-5-yl)méthyl]-5-(2-fluoroéthyl)-4-méthylthiazolium ou un sel de celui-ci, où l'élément de fluor dans le fluoroéthyle sur le noyau thiazole est le radioisotope ¹⁸F, présentant une structure chimique de formule (I) ci-dessous :

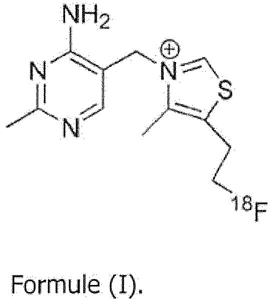

2. Thiamine substituée par le radioisotope ¹⁸F ou sel de celle-ci selon la revendication 1, présentant une structure chimique de formule (II) ou (III) ci-dessous :

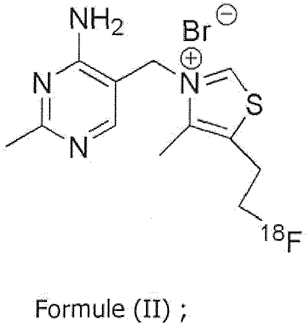

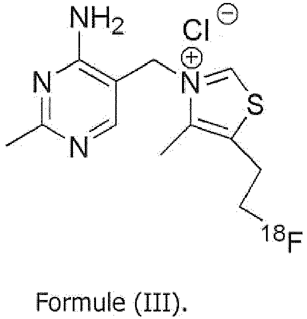

3. Thiamine substituée par le radioisotope ¹⁸F ou sel de celle-ci selon la revendication 2, dans laquelle le sel de la thiamine substituée par le radiosotope ¹⁸F est spécifiquement un hydrobromure de thiamine substituée par le radioisotope ¹⁸F présentant une formule chimique de C₁₂H₁₆¹⁸FN₄SBrHBr, et une structure chimique de formule (IV) ci-dessous :

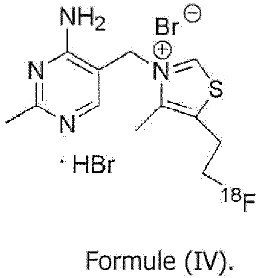

4. Procédé de synthèse d'un hydrobromure de thiamine substituée par le radioisotope ¹⁸F selon la revendication 3, comprenant :
Etape 1) : production de protons H⁺ dans un cyclotron, bombardement de ¹⁸O-eau lourde avec les protons H⁺, et production d'ions ¹⁸F⁻ par réaction nucléaire ¹⁸O(p, n)¹⁸F ; enrichissement d'ions ¹⁸F⁻ par une colonne QMA, et élution de ceux-ci dans une fiole de réaction avec un éluant, pour obtenir [K/Kryptofix]⁺¹⁸F⁻, évaporation d'eau jusqu'à l'état sec ; et réaction de 4-méthylbenzènesulfonate de 2-(4-méthylthiazol-5-yl)éthyle avec [K/Kryptofix]⁺¹⁸F⁻ pour produire du 5-(2-fluoroéthyl)-4-méthylthiazole par substitution nucléophile dans un solvant d'acétonitrile, où l'équation de réaction pour la synthèse chimique est : 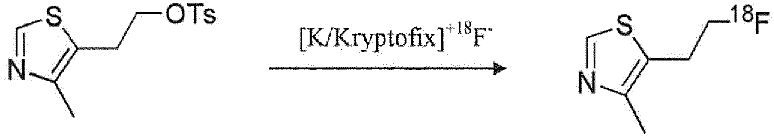
Etape 2) : réaction du produit de 5-(2-fluoroéthyl)-4-méthylthiazole obtenu dans l'étape 1) avec de l'hydrobromure de 2-méthyl-5-bromométhyl-6-aminopyrimidine dans un solvant d'acétonitrile pour produire de l'hydrobromure de thiamine substituée par ¹⁸F, où l'équation de réaction pour la synthèse chimique est :

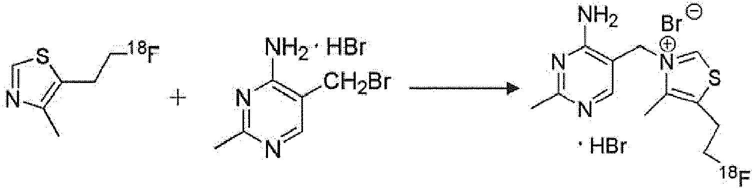

5. Procédé de synthèse d'hydrobromure de thiamine substituée par le radioisotope ¹⁸F selon la revendication 4, dans lequel le rapport de 4-méthylbenzènesulfonate de 2-(4-méthylthiazol-5-yl)éthyle au solvant dans l'étape 1) est de 5 mg:0,5-1 ml ; le rapport de 4-méthylbenzènesulfonate de 2-(4-méthylthioazol-5-yl)éthyle aux ions ¹⁸F⁻ est de 5 mg:4-6 Ci ; et le rapport de 4-méthylbenzènesulfonate de 2-(4-méthylthiazol-5-yl)éthyle à l'éluant est de 5 mg:0,6-0,9 ml, où chaque 0,9 ml de l'éluant contient 2,52 mg de KHCO₃, 9 mg de Kryptofix 2.2.2, 0,72 ml d'acétonitrile, et 0,18 ml d'eau.

6. Procédé de synthèse d'hydrobromure de thiamine substituée par le radioisotope ¹⁸F selon la revendication 4, dans lequel le rapport d'hydrobromure de 2-méthyl-5-bromométhyl-5-aminopyrimidine au solvant dans l'étape 2) est de 5 mg:0,5-1 ml.

7. Procédé de synthèse d'hydrobromure de thiamine substituée par le radioisotope ¹⁸F selon l'une quelconque des revendications 4 à 6, dans lequel la température de réaction dans les étapes 1) et 2) est de 110°C.

8. Procédé de synthèse d'hydrobromure de thiamine substituée par le radioisotope ¹⁸F selon la revendication 4, dans lequel le rapport massique de 4-méthylbenzènesulfonate de 2-(4-méthylthiazol-5-yl)éthyle dans l'étape 1) à l'hydrobromure de 2-méthyl-5-bromométhyl-6-aminopyrimidine dans l'étape 2) est de 1:1.

9. Utilisation de la thiamine substituée par le radioisotope ¹⁸F selon la revendication 1 dans la préparation d'un traçeur pour la formation d'image PET du cerveau chez les rongeurs.

10. Utilisation de la thiamine substituée par le radioisotope ¹⁸F selon la revendication 1 dans la préparation d'un traçeur pour la formation d'image PET d'essais cliniques pour la maladie d'Alzheimer.

11. Utilisation de la thiamine substituée par un radioisotope ¹⁸F selon la revendication 1 dans la préparation d'un traçeur pour la formation d'image PET d'essais cliniques pour des tumeurs.
